(19) Europäisches Patentamt
European Patent Office
Office européen des brevets

(11) **EP 2 379 033 B1**

(12) **EUROPÄISCHE PATENTSCHRIFT**

(45) Veröffentlichungstag und Bekanntmachung des Hinweises auf die Patenterteilung:
**18.09.2013 Patentblatt 2013/38**

(51) Int Cl.:
***A61F 13/00*** *(2006.01)*

(21) Anmeldenummer: 09796970.3

(22) Anmeldetag: **21.12.2009**

(86) Internationale Anmeldenummer:
**PCT/EP2009/009190**

(87) Internationale Veröffentlichungsnummer:
**WO 2010/072395 (01.07.2010 Gazette 2010/26)**

(54) **WUNDAUFLAGE**

WOUND DRESSING

PANSEMENT

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **23.12.2008 DE 102008062824**

(43) Veröffentlichungstag der Anmeldung:
**26.10.2011 Patentblatt 2011/43**

(73) Patentinhaber: **Paul Hartmann AG 89522 Heidenheim (DE)**

(72) Erfinder:
• **HOFSTETTER, Jürgen**
  **89522 Heidenheim (DE)**
• **MANES, Albert**
  **E-08005 Barcelona (ES)**

(56) Entgegenhaltungen:
**EP-A1- 1 923 077       WO-A1-2004/112852**
**GB-A- 2 272 645       US-A1- 2005 165 372**
**US-A1- 2007 293 799**

**Beschreibung**

Die vorliegende Erfindung betrifft Wundauflagen und deren Verwendung in der modernen Wundbehandlung. Dabei betrifft die vorliegende Erfindung Wundauflagen, die zwei voneinander verschiedene absorbierende Schichten, insbesondere zwei unterschiedlich stark absorbierende Schichten, zur Behandlung von mäßig bis stark exsudierenden Wunden umfasst.

Im Zusammenhang mit der Heilung von Wunden, insbesondere von Wunden, die durch operative Eingriffe verursacht sind, sind in der Vergangenheit eine Vielzahl an Produkten dem Markt zur Verfügung gestellt worden. Dazu ist der Fachliteratur als auch der Patentliteratur eine Vielzahl an Vorschlägen zu entnehmen, wie und womit eine Wunde zur Heilung gebracht werden kann.

So wird beispielsweise mit der deutschen Anmeldung DE-19727032-A1 ein Wundverband zur postoperativen Anwendung beschrieben, der zwei unterschiedlich stark haftende Klebebereiche um ein absorbierendes Kissen aufweist. Als absorbierende Kissen können hierbei insbesondere Faservliese verwendet werden. Die unterschiedlich stark haftenden Klebebereiche ermöglichen ein leichteres Entfernen von der Haut, wobei ein Schädigen von vorgeschädigter Haut unterbleibt.

Mit den beiden US- amerikanischen Patenten US- 5, 465, 735- A und US- 5, 632, 731- A werden nicht mit einer Wunde verklebende Wundauflagen beschrieben, die eine Trägerschicht und ein absorbierendes Kissen umfassen. Die absorbierenden Kissen sind mehrlagig aufgebaut, wobei jedes Kissen zwei verschiedene Faservliese mit unterschiedlicher Faserdichte umfasst, die wundseitig mit einer perforierten, nicht- wundverklebenden Folie abgedeckt sind. Durch die Verwendung einer nicht- wundverklebenden Folie wird ein traumatisches Entfernen von der Wunde vermieden.

Mit der internationalen Anmeldung WO-98/41095-A1 wird eine mehrlagige, antimikrobielle Wundauflage beschrieben, die eine Wundkontaktschicht, eine absorbierende Schicht und eine Abdeckschicht aufweist. Hierbei wird als Wundkontaktschicht eine Silberbeschichtung aufweisende Polymerfolie verwendet, die Silber mit einer atomaren Unordnung aufweist. Hierbei kann zusätzlich vorgesehen sein, dass weitere Schichten ebenfalls mit Silber beschichtet sind. Durch die Verwendung von Silber mit einer atomaren Unordnung soll im Vergleich zu Silber ohne atomare Unordnung in Gegenwart von Elektrolyten vermehrt Silber freigesetzt werden.

Weiterhin werden mit der internationalen Anmeldungen WO-01/60599-A1 mehrschichtige antimikrobielle Wundauflagen beschrieben, die eine Wundkontaktschicht aus einem mit Silber beschichteten Textilmaterial wie z.B. ein mit Silber beschichtetes Gewebe oder Nonwoven umfassen, das mit einem Gel beschichtet ist. Wesentlich ist hierbei, dass Silber in Form von Ionen zum Einsatz gegen Keime in einer Wunde von der Wundauflage in die Wunde abgegeben werden.

Darüber hinaus ist mit der internationalen Anmeldung WO-2004/112852-A1 eine antimikrobielle Wundauflage beschrieben, die eine einseitig mit Silber beschichtete Wundkontaktfolie umfasst. Die mit Silber beschichtete Seite liegt dabei zu einer absorbierenden Schicht zugewandt. Durch diese Anordnung soll eine antimikrobielle Wundauflage bereitgestellt werden, die Silber in einer zur Keimtötung ausreichenden Menge an eine Wunde abgibt, wobei keine Verfärbung durch Silberabrieb auftritt.

Weiterhin wird mit der deutschen Anmeldung DE-10108083-A1 eine geruchsadsorbierende Wundauflage beschrieben, die als animikrobielles Mittel Silber enthält. Das antimikrobielle Mittel ist dabei zwingend in einer von der geruchsadsorbierenden Schicht verschiedenen Schicht ein- oder aufgebracht. Hierdurch soll eine Wundauflage mit verbesserter Geruchsneutralisation bereitgestellt werden, wobei die Wundauflage gleichzeitig Silber abgeben und somit Keime in einer Wunde abtöten kann.

Die somit beschriebenen Wundauflagen weisen insgesamt den Nachteil auf, dass die Wundauflagen in ihrer bestimmungsgemäßen Verwendung eine sehr hohe Konzentration an Silber in die Wunde abgeben.

Daher liegt der vorliegenden Erfindung die Aufgabe zugrunde, eine Wundauflage mit verbesserten Absorptionseigenschaften bereitzustellen, die auch bei längerer Verwendung auf einer mäßig bis stark exsudierenden Wunde eine Kontamination mit Keimen entgegenwirkt, wobei die Wundauflage die zu behandelnde Wunde nicht mit überschüssigen Wirkstoffen belastet. Darüber hinaus soll eine Wundauflage bereitgestellt werden, die der Mazeration der Wunde und der umliegenden Hautareale entgegenwirkt. Gemäß einem weiteren Aspekt soll auch eine Wundauflage bereitgestellt werden, die bei längerer Verwendung die Gefahr des Verklebens mit der Wunde reduziert und die gleichzeitig der Mazeration entgegenwirkt.

Gelöst werden diese Aufgaben durch eine Wundauflage gemäß den Merkmalen des Anspruchs 1. Demnach umfasst eine erfindungsgemäße Wundauflage eine Trägerschicht und ein absorbierendes Kissen, wobei das absorbierende Kissen eine erste absorbierende Schicht umfassend ein erstes absorbierendes Material und eine von der ersten Schicht verschiedene zweite absorbierende Schicht umfassend ein von dem ersten Material verschiedenes zweites absorbierendes Material umfasst, und wobei die erste Schicht von der zweiten Schicht durch mindestens eine dritte Schicht beabstandet ist, wobei diese dritte Schicht mindestens ein antimikrobielles Mittel umfasst, wobei das zweite absorbierende Material superabsorbierende Fasern umfasst und wobei das absorbierende Kissen weiterhin eine Wundkontaktschicht umfasst, die mit der ersten Schicht in unmittelbarem Kontakt steht, wobei die Wundkontaktschicht ein Polymernetz oder eine perforierte Polymerfolie ist.

Hierbei ist im Zusammenhang mit der vorliegenden Erfindung unter Absorption immer und ausschließlich die Aufnahme von Flüssigkeiten zu verstehen. Des Weiteren ist im Zusammenhang mit der vorliegenden Erfindung unter einer Beabstandung die räumliche Trennung von zwei Komponenten zu verstehen, wobei diese räumliche Trennung vollständig oder lediglich in Teilen erfolgt sein kann; d.h., dass die beiden beabstandeten Komponenten in Teilbereichen beabstandet und in weiteren Teilbereichen in direktem Kontakt stehen können.

Somit kann eine Wundauflage bereitgestellt werden, die ein antimikrobielles Mittel umfasst, das bei der Anwendung der Wundauflage nicht direkt mit einer Wunde in Kontakt kommt, wodurch eine Überbelastung an antimikrobiellem Mittel in einer Wunde unterbleibt. Darüber hinaus wird mit der erfindungsgemäßen Anordnung der Schichten eine Wundauflage bereitgestellt, dessen antimikrobielles Mittel fast ausschließlich innerhalb des absorbierenden Kissens seine Wirkung entfaltet. Des Weiteren wird eine Wundauflage bereitgestellt, die abhängig vom aufkommenden Wundexsudatfluss vermehrtes Wundexsudat aufnimmt somit absorbiert und von der Wunde fernhält. Hierdurch werden eventuell vorhandenen Bakterien der Nährboden entzogen.

Die zweite absorbierende Schicht ist zwischen der ersten absorbierenden Schicht und der Trägerschicht angeordnet. Gleichzeitig oder unabhängig hiervon kann dabei vorgesehen sein, dass die erste absorbierende Schicht als Flüssigkeitsverteilerschicht und die zweite absorbierende Schicht als Flüssigkeitsdepotschicht ausgebildet ist. Beide absorbierende Schichten weisen jedoch absorbierende Eigenschaften auf. Insbesondere umfasst eine erfindungsgemäße Wundauflage jedoch eine erste und eine zweite absorbierende Schicht, die vor der bestimmungsgemäßen Verwendung keine antimikrobiellen Mittel umfassen.

Als absorbierende Materialien können gemäß der vorliegenden Erfindung eine Vielzahl an in der Wundversorgung bekannten Materialien verwendet werden. Insbesondere ist hierbei vorgesehen sowohl die erste als auch die zweite absorbierende Schicht aus Fasermaterialien zu fertigen. Dabei hat sich weiterhin als vorteilhaft erwiesen, wenn das absorbierende Kissen eine erste absorbierende Schicht und eine zweite absorbierende Schicht umfasst, wobei die zweite absorbierende Schicht eine größere freie Absorption aufweist als die erste absorbierende Schicht, wobei die freie Absorption gemäß DIN EN 13726-1 (2002) bestimmt wird. Gleichzeitig oder unabhängig hiervon ist hierbei weiterhin vorteilhaft vorgesehen, dass die erste absorbierende Schicht im anwendungsgerechten Zustand der Wundauflage zur Wunde weist. In der bestimmungsgemäßen Verwendung der Wundauflage wird somit das Wundexsudat von der Wunde abgehalten, wobei die erste absorbierende Schicht das Wundexsudat unidirektional zu der zweiten Schicht transportiert und diese zweite absorbierende Schicht das Wundexsudat speichert. Somit kann auch eine Wundauflage bereit gestellt werden, die das Wundexsudat besonders gut aufnimmt, wobei die Wundoberfläche weitgehenst trocken gehalten wird und eine Mazeration unterbunden wird. Durch den unidirektionalen Transport des Wundexsudats kommt das Wundexsudat auch in direkten Kontakt mit dem antimikrobiellen Mittel der dritten Schicht, wobei diese ihre antimikrobielle Wirkung entfalten. So kann die Wundauflage auch über einen längeren Zeitraum auf einer Wunde verbleiben, ohne dass eine Rekontamination mit Keimen durch die Wundauflage erfolgt.

Insbesondere ist dabei auch vorgesehen, dass die erste absorbierende Schicht eine freie Absorption $A_1$ von mindestens 0,5 g/ g, insbesondere mindestens 2 g/ g und ganz besonders bevorzugt von mindestens 4 g/ g aufweist, wobei die freie Absorption $A_1$ gemäß der DIN-EN 13726-1 (2002) bestimmt wird. Hierbei ist ganz besonders bevorzugt vorgesehen, dass die erste absorbierende Schicht eine freie Absorption $A_1$ von höchstens 10 g/ g aufweist. Ganz besonders bevorzugt ist dabei, dass die erste absorbierende Schicht eine freie Absorption $A_1$ von mindestens 1 g/ g bis 10 g/ g, insbesondere von mindestens 2 g/ g bis 10 g/ g und ganz besonders bevorzugt von mindestens 5 g/ g bis 10 g/ g aufweist.

Des Weiteren ist gleichzeitig oder unabhängig hiervon bevorzugt vorgesehen, dass die zweite absorbierende Schicht eine freie Absorption $A_2$ von mindestens 12 g/ g, insbesondere mindestens 15 g/ g und ganz besonders bevorzugt von mindestens 20 g/ g aufweist, wobei die freie Absorption $A_2$ gemäß der DIN-EN 13726-1 (2002) bestimmt wird. Hierbei ist ganz besonders bevorzugt vorgesehen, dass die zweite absorbierende Schicht eine freie Absorption $A_2$ von höchstens 50 g/ g aufweist. Ganz besonders bevorzugt ist dabei, dass die zweite absorbierende Schicht eine freie Absorption $A_2$ von mindestens 12 g/ g bis 50 g/ g, insbesondere von mindestens 15 g/ g bis 50 g/ g und ganz besonders bevorzugt von mindestens 20 g/ g bis 50 g/ g aufweist.

Gleichzeitig oder unabhängig hiervon weist eine erfindungsgemäße Wundauflage ein absorbierendes Kissen auf, das eine freie Absorption $A_T$ von mindestens 8 g/ g insbesondere von mindestens 12 g/ g und ganz besonders bevorzugt von mindestens 20 g/ g , wobei die freie Absorption $A_T$ gemäß DIN-EN 13726-1 (2002) bestimmt wird. Hierbei ist weiterhin bevorzugt vorgesehen, dass das absorbierende Kissen eine freie Absorption $A_T$ von höchstens 50 g/ g aufweist. Ganz besonders bevorzugt ist dabei, dass das absorbierende Kissen eine freie Absorption $A_T$ von mindestens 8 g/ g bis 50 g/ g, insbesondere von mindestens 12 g/ g bis 50 g/ g und ganz besonders bevorzugt von mindestens 20 g/ g bis 50 g/ g aufweist.

Weiterhin gleichzeitig oder unabhängig voneinander weist eine erfindungsgemäße Wundauflage eine erste absorbierende Schicht und eine von der ersten absorbierenden Schicht verschiedene zweite absorbierende Schicht auf, deren freie Absorptionen $A_1$ und $A_2$ derart eingestellt ist, dass das Verhältnis $A_1 : A_2$ dem Verhältnis von 1 : 2 bis 1 : 10 entspricht. Besonders bevorzugt entspricht das Verhältnis $A_1$ zu $A_2$ dem Verhältnis von 1 : 3 bis 1 : 10 und ganz besonders bevorzugt 1 : 5 bis 1 : 10.

Gemäß einer weiteren Ausführung der vorliegenden Erfindung ist dabei insbesondere vorgesehen, dass das erste absorbierende Material Cellulosefasern oder Viskosefasern umfasst. Insbesondere kann hierbei auch vorgesehen sein, dass das erste absorbierende Material ein Faservlies umfasst, das Viskosefasern und thermoplastischen Fasern umfasst, besonders bevorzugt daraus besteht, wobei als thermoplastische Fasern weiterhin bevorzugt Polypropylen- oder Polyethylenfasern verwendet werden können. Diese thermoplastischen Fasern können auch als so genannte Bikomponentenfasern in Form von Mantel-Kern-Fasern vorliegen.

Das zweite absorbierende Material umfasst superabsorbierende Fasern. Diese Fasern weisen eine besonders hohe freie Absorption auf und weisen im Unterschied zu partikulären superabsorbierenden Materialien den Vorteil auf, dass sie besonders gut mit anderen Fasern durchmischt und festgehalten werden können. Somit kann bei Verwendung von superabsorbierenden Fasern auf eine Umhüllung der zweiten absorbierenden Schicht verzichtet werden.

Insbesondere ist hierbei vorgesehen, dass die zweite absorbierende Schicht als absorbierendes Material ein Faservlies umfasst, das ein Fasergemisch aus superabsorbierenden Fasern und Stützfasern umfasst. Hierbei kann jedoch auch vorgesehen sein, dass das Faservlies superabsorbierenden Fasern und Stützfasern umfasst und die Stützfasern ausgewählt werden aus der Gruppe der thermoplastische Fasern und/ oder Cellulosefasern und/ oder Viskosefasern. Ganz besonders bevorzugt umfasst das Fasergemisch superabsorbierende Fasern, Viskosefasern und thermoplastische Fasern wobei als thermoplastische Fasern weiterhin bevorzugt Polypropylen- oder Polyethylenfasern verwendet werden können. Diese thermoplastischen Fasern können auch als so genannte Bikomponentenfasern in Form von Mantel-Kern-Fasern vorliegen.

Dabei enthält das zweite absorbierende Material vorzugsweise ein Fasergemisch, das mehr als 20 Gew.-%, insbesondere mehr als 30 Gew.-%, bevorzugt mehr als 40 Gew.-%, bevorzugt mehr als 50 Gew.-% und besonders bevorzugt mehr als 60 Gew.-% superabsorbierende Fasern umfasst. Hierdurch kann ein absorbierendes Kissen bereitgestellt werden, das eine besonders hohe freie Absorption aufweist. Eine entsprechend ausgerüstete Wundauflage kann bis zu drei und mehr Tagen auf einer Wunde verbleiben.

Die Bezeichnung "superabsorbierend" bezieht sich dabei auf ein in Wasser quellbares, im wesentlichen wasserunlösliches Material in Form von Fasern, das in der Lage ist, mindestens das ca. 10- fache, vorzugsweise ca. 20-fache und besonders bevorzugt ca. 50- fache oder mehr seines Gewichts an Wasser zu absorbieren. Das superabsorbierende Material kann dabei aus einem organischen Material gebildet sein, das synthetisches und/ oder natürliches Material umfassen kann, wie beispielsweise Agar, Pektin und Guargummi oder auch synthetische Materialien, wie beispielsweise synthetische Hydrogelpolymere. Synthetische Hydrogelpolymere umfassen beispielsweise Carboxymethylcellulose, Alkalimetallsalze von Polyacrylsäuren, Alkalimetallsalze von Polymethacrylsäuren, Polyacrylamide, Polyvinylalkohol, Ethylenmaleinsaeureanhydrid- Copolymere, Polyvinylether, Hydroxypropylcellulose, Polymere und Copolymere von Vinyl- Sulfonsäure, Polyacrylate, Polymethacrylate, Polyacrylat- Polymethacrylat- Copolymere, Polyacrylamide und Ähnliche. Ganz besonders bevorzugt sind als superabsorbierende Fasern solche Fasern, die aus Alkalimetallsalzen von Polyacrylsäuren, Alkalimetallsalzen von Polymethacrylsäuren, Polyacrylaten, Polymethyacrylaten oder Polyacrylat- Polymethacrylat- Copolymeren hergestellt sind. Die Hydrogelpolymere sind vorzugsweise leicht vernetzt um die Materialien im Wesentlichen wasserunlöslich zu machen. Die Vernetzung kann z. B. durch Bestrahlung oder kovalente, ionische, von van- der- Waals- oder Wasserstoffbrückenbindung geschehen. Geeignete Materialien sind von verschiedenen Herstellern wie BASF und Stockhausen Inc. erhältlich.

Weiterhin bevorzugt ist hierbei vorgesehen, dass die zweite absorbierende Schicht als absorbierendes Material ein Faservlies umfasst, das ein Fasergemisch aus mindestens 20 Gew.% superabsorbierenden Fasern, mindestens 10 Gew.-% Viskose- oder Cellulosefasern und mindestens 5 Gew.-% thermoplastische Fasern umfasst. Ganz besonders bevorzugt ist vorgesehen, dass die zweite absorbierende Schicht als absorbierendes Material ein Faservlies umfasst, das ein Fasergemisch aus mindestens 40 Gew.-% superabsorbierenden Fasern, mindestens 20 Gew.-% Viskose- oder Cellulosefasern und mindestens 5 Gew.-% thermoplastische Fasern umfasst. Durch den Anteil an Stützfasern in dem Faservlies erfolgt zu Beginn der Absorption eine gleichmäßige Verteilung der aufzunehmenden Flüssigkeitsmenge.

Als besonders vorteilhafte Ausführungen haben sich weiterhin Wundauflagen gezeigt, die ein absorbierendes Kissen umfassen, dessen Schichtdicke eine Schichtdicke S von 0,1 bis 10,0 mm aufweist. Insbesondere weist eine erfindungsgemäße Wundauflage ein absorbierendes Kissen auf, das eine Schichtdicke S von 0,1 bis 8,0 mm, insbesondere von 0,1 bis 7,0 mm und ganz besonders bevorzugt von 0,5 bis 5,0 mm aufweist. Wundauflagen mit solchen Schichtdicken zeigen einerseits die Fähigkeit ein von einer Wunde abgegebenes Wundexsudat nachhaltig aufzunehmen zu können und gleichzeitig eine gute Drapierfähigkeit zu gewährleisten. Diese Schichtdicken können an jeder Stelle der des absorbierenden Kissens gleich sein oder in verschiedenen Bereichen des absorbierenden Kissens verschiedene Werte annehmen.

Gemäß der vorliegenden Erfindung sind die erste und die zweite absorbierende Schicht durch eine dritte Schicht beabstandet, wobei die dritte Schicht ein antimikrobielles Mittel umfasst. Diese dritte Schicht stellt somit ein antimikrobielles Mittel zur Verfügung, das innerhalb der Wundauflage seine Wirkung entfaltet und Keime innerhalb der Wundauflage abtötet. Dabei hat es sich als vorteilhaft gezeigt, wenn als dritte Schicht eine im Vergleich zu der ersten oder der zweiten absorbierenden Schicht vergleichbar dünnere dritte Schicht verwendet wird.

In Weiterbildung des Erfindungsgedankens hat es sich als vorteilhaft erwiesen, wenn die Wundauflage als dritte Schicht ein Polymernetz, eine perforierte Polymerfolie, ein Faservlies oder ein textiles Material wie Gestrick, Gewirk oder Gewebe umfasst. Dabei kann insbesondere auch vorgesehen sein, dass die dritte Schicht aus einem Polymernetz oder einer perforierten Polymerfolie besteht, das/ die eine Vielzahl an Löcher, Öffnungen oder Durchbrechungen oder Kanälen mit einem durchschnittlichen Durchmesser von 0,01 mm bis 1 mm, vorzugsweise von 0,1 mm bis 1 mm zum Durchtritt von Flüssigkeiten, insbesondere Wundexsudat aufweist.

Weiterhin bevorzugt kann die dritte Schicht eine Vielzahl an Löcher, Öffnungen, Durchbrechungen oder Kanälen zum Durchtritt von Flüssigkeiten aufweisen, wobei diese Löcher, Öffnungen, Durchbrechungen oder Kanäle eine Fläche von mindestens 20 % der Fläche der dritten Schicht einnehmen. Hierbei ist weiterhin bevorzugt vorgesehen, dass die Löcher, Öffnungen, Durchbrechungen oder Kanäle eine Fläche von mindestens 25 %, insbesondere mindestens 30 % und ganz besonders bevorzugt mindestens 35 % der Fläche der dritten Schicht einnehmen. Ganz besonders bevorzugt weist die dritte Schicht Löcher, Öffnungen, Durchbrechungen oder Kanäle auf, die eine Fläche von mindestens 20 % und höchstens 80 % der Fläche der dritten Schicht einnehmen. Ganz besonders bevorzugt weist die Wundauflage eine dritte Schicht auf, die 10 bis 100 Löcher pro cm$^2$ aufweist. Die dritte Schicht ist ein Polymernetz oder eine perforierte Polymerfolie, insbesondere aus Polyethylen oder Polypropylen. Somit wird die erste absorbierende Schicht von der zweiten absorbierenden Schicht in ausreichendem Maß voneinander beabstandet und gleichzeitig eine ausreichende Menge an antimikrobiellen Mitteln innerhalb der Wundauflage bereitgestellt.

Hierbei ist insbesondere vorgesehen, dass das antimikrobielle Mittel direkt auf die dritte Schicht aufgebracht oder beschichtet ist. Es kann jedoch auch vorgesehen sein, dass die dritte Schicht mit dem antimikrobiellen Mittel imprägniert ist oder dass das antimikrobielle Mittel in der dritten Schicht inkorporiert ist.

In besonders vorteilhafter Weise wird in einer weiteren Ausführung als dritte Schicht ein Polymernetz oder eine perforierte Polymerfolie, ein Nonwoven oder ein textiles Material wie Gestrick, Gewirk oder Gewebe verwendet, das mit Silber beschichtet oder imprägniert ist. Es kann jedoch auch vorgesehen sein, dass das Silber in die dritte Schicht inkorporiert ist. Gleichzeitig oder unabhängig hiervon kann dabei insbesondere vorgesehen sein, dass das Silber in Form von elementarem Silber, einem Silberoxid, einem Silbersalz oder einem Silberkomplex oder Mischungen hiervon verwendet wird. Damit umfasst eine erfindungsgemäße Wundauflage als antimikrobielles Mittel insbesondere elementares Silber, Silberoxid oder Silber in Form von Silberkomplexen oder Silbersalzen. Ganz besonders bevorzugt ist hierbei vorgesehen, als antimikrobielles Mittel elementares Silber zu verwenden. Silber weist insbesondere in seiner elementaren Form ein sehr niedriges Löslichkeitsprodukt auf, wodurch im Vergleich zu anderen antimikrobiellen Wirkstoffen eine in Gegenwart von Wundexsudat sehr niedrige Konzentration an Wirkstoff freigesetzt wird. Diese Konzentration reicht jedoch aus, um eine antimikrobielle Wirkung innerhalb der Wundauflage bereitzustellen. Ohne an die Theorie gebunden zu sein, entfaltet das Silber innerhalb einer erfindungsgemäßen Wundauflage gebunden in elementarer Form auf der dritten Schicht und in Form von durch das Wundexsudat aus dem elementaren Verbund ausgelösten Silberionen oder Silberkomplexionen seine Wirkung. Diese Ionen haben jedoch die Tendenz mit entgegengesetzt geladenen Ionen in Wechselwirkung zu treten. Im Fall von positiv geladenen Silberionen und dem Einsatz von superabsorbierende Fasern aus Salzen der Polyacrylsäure oder Polyacrylate, die bekanntermaßen negativ geladene Regionen aufweisen, werden selbst die durch das Wundexsudat freigesetzten Silberionen oder Silberkomplexionen durch diese Fasern innerhalb der Wundauflage gehalten. Somit kann das Silber bis auf eine sehr geringe Konzentration lediglich innerhalb der Wundauflage wirken, wodurch auch eine Kontamination mit zu hohen Mengen an Silber auf der Wunde verhindert wird.

Gemäß einem weiterführenden Gedanken setzt das absorbierende Kissen weniger als 25 $\mu$g Silber pro 1 g absorbierendes Kissen in 24 Std. bei 25 °C in 100 ml Wasser oder physiologischer Kochsalzlösung, insbesondere weniger als 15 $\mu$g Silber pro 1 g absorbierendes Kissen in 24 Std. bei 25 °C in 100 ml Wasser oder physiologischer Kochsalzlösung und insbesondere weniger als weniger als 5 $\mu$g Silber pro 1 g absorbierendes Kissen in 24 Std. bei 25 °C in 100 ml Wasser oder physiologischer Kochsalzlösung an Silber in elementarer Form oder in Form von Salzen oder Komplexen frei. Zur Bestimmung der abgegebenen Menge Silber wird die Atomabsorptionsspektroskopie herangezogen.

Gemäß einer weiteren Ausgestaltung der vorliegenden Erfindung kann eine erfindungsgemäße Wundauflage mindestens eine oder mehrere weitere Schichten umfassen, die in dem Gesamtsystem Wundauflage verschiedene Funktionen erfüllen. Es ist vorgesehen, dass das absorbierende Kissen weiterhin eine Wundkontaktschicht umfasst, die mit der ersten Schicht in unmittelbaren Kontakt steht. Ganz besonders bevorzugt ist hierbei vorgesehen, dass diese Wundkontaktschicht vor der bestimmungsgemäßen Verwendung der Wundauflage keine antimikrobiellen Mittel umfasst. Somit kann eine antimikrobielle Wundauflage bereitgestellt werden, die ein antimikrobielles Mittel umfasst, das lediglich im Inneren der Wundauflage wirkt.

Gemäß einer weiteren Ausbildung der vorliegenden Erfindung kann auch vorgesehen sein, dass die Wundauflage eine Wundkontaktschicht umfasst, die dieselbe Auflagefläche aufweist wie die Trägerschicht. Hierbei kann vorgesehen sein, dass die Wundkontaktschicht im Bereich des absorbierenden Kissens in direktem Kontakt mit der ersten oder der zweiten Schicht steht und im Randbereich der Wundauflage mit der Trägerschicht verbunden ist. In dieser Ausgestaltung bildet die Wundkontaktschicht mit der Trägerschicht zusammen eine Umhüllung für das absorbierende Kissen.

Die Wundkontaktschicht ist ein Polymernetz oder eine perforierte Polymerfolie. Diese Polymernetze oder perforierten Folien weisen den Vorteil auf, dass sie nicht mit einer Wunde verkleben. Somit wird durch Verwendung einer solchen Folie oder eines solchen Netzes auch eine nicht wundverklebende Wundauflage bereitgestellt.

Dabei kann insbesondere auch vorgesehen sein, dass die Wundkontaktschicht aus einem Polymernetz oder einer perforierten Polymerfolie besteht, das/die eine Vielzahl an Löcher, Öffnungen oder Durchbrechungen oder Kanälen mit einem durchschnittlichen Durchmesser von 0,01 mm bis 1 mm, vorzugsweise von 0,1 mm bis 1 mm zum Durchtritt von Flüssigkeiten, insbesondere Wundexsudat aufweist.

Weiterhin bevorzugt kann die Wundkontaktschicht eine Vielzahl an Löcher, Öffnungen, Durchbrechungen oder Kanälen zum Durchtritt von Flüssigkeiten aufweisen, wobei diese Löcher, Öffnungen, Durchbrechungen oder Kanäle eine Fläche von höchstens 30 % der Fläche der Wundkontaktschicht einnehmen. Hierbei ist weiterhin bevorzugt vorgesehen, dass die Löcher, Öffnungen, Durchbrechungen oder Kanäle eine Fläche von höchstens 25 %, insbesondere höchstens 20 %, insbesondere höchstens 15 % und ganz besonders bevorzugt von höchstens 10 % der Fläche der Wundkontaktschicht einnehmen. Ganz besonders bevorzugt weist die Wundkontaktschicht Löcher, Öffnungen, Durchbrechungen oder Kanäle auf, die eine Fläche von mindestens 10 % und höchstens 20 % der Fläche der ersten Seite der Wundkontaktschicht einnehmen. Ganz besonders bevorzugt weist die Wundauflage eine Wundkontaktschicht auf, die 10 bis 100 Löcher pro cm$^2$ aufweist. Hierbei kann vorgesehen sein, dass die Wundkontaktschicht ein gelochter Polymerfilm oder ein Polymernetz, insbesondere aus Polyethylen oder Polypropylen ist.

Gemäß einer weiteren bevorzugten Ausbildung der Erfindung umfasst die Wundauflage als Wundkontaktschicht ein Polymernetz oder eine perforierte Polymerfolie aus Polyethylen oder Polypropylen, und eine erste absorbierende Schicht mit einem ersten absorbierenden Material, welches ein Faservlies umfasst. Dabei umfasst das Faservlies 80 bis 99 Gew. % Viskosefasern und 1 bis 20 Gew. % thermoplastische Fasern, wobei die thermoplastischen Fasern vorzugsweise Polypropylen- oder Polyethylenfasern sind, welche als so genannte Bikomponentenfasern in Form von Mantel-Kern-Fasern vorliegen. Besonders bevorzugt enthält das Faservlies gemäß dieser Ausbildung der Erfindung 85 Gew. % Viskosefasern und 15 Gew. % Bikomponentenfasern in Form von Mantel-Kern-Fasern. Der Anteil an Bikomponentenfasern im Faservlies kann die Haftung zwischen der ersten absorbierenden Schicht und der Wundkontaktschicht verstärken.

Als Trägerschicht kann gemäß der vorliegenden Erfindung jedes heute bekannte Trägermaterial eingesetzt werden, das eine ausreichende Reißfestigkeit aufweist und weitgehenst undurchlässig für Flüssigkeiten ist. Als Trägerschicht einer erfindungsgemäßen Wundauflage können dabei insbesondere Faservliese oder Polymerfilme eingesetzt werden. Weiterhin bevorzugt können als Trägerschicht einer erfindungsgemäßen Wundauflage insbesondere Polymerfilme eingesetzt werden, die wasserundurchlässig sind und die eine hohe Wasserdampfdurchlässigkeit aufweisen. Hierzu sind besonders Filme geeignet, die aus Polyurethan, Polyetherurethan, Polyesterurethan, Polyether-Polyamid-Copolymeren gefertigt werden. Insbesondere ist als Trägerschicht ein wasserundurchlässiger und wasserdampfdurchlässiger Polyurethanfilm, Polyesterurethanfilm oder Polyetherurethanfilm bevorzugt. Ganz besonders sind auch solche Polymerfilme bevorzugt, die eine Dicke von 15 bis 50 μm, insbesondere 20 bis 40 μm und ganz besonders bevorzugt von 25 bis 30 μm aufweisen. Die Wasserdampfdurchlässigkeit des Polymerfilms der Wundauflage weist vorzugsweise mindestens 750 g/ m$^2$/ 24 Std., insbesondere mindestens 1000 g/ m$^2$/ 24 Std. und ganz besonders bevorzugt mindestens 2000 g/ m$^2$/ 24 Std. auf (gemessen nach DIN EN 13726-2). In besonders bevorzugten Ausführungsformen weisen diese Filme einen feuchtigkeitsdichten, klebenden Randabschnitt auf. Dieser Randabschnitt gewährleistet, dass die Wundauflage an seinem bestimmungsgemäßen Ort appliziert und fixiert werden kann. Als besonders bevorzugt sind solche Klebstoffe zu betrachten, die in einem dünnen Auftrag von 20 bis 35 g/ m$^2$ zusammen mit dem Film eine Wasserdampfdurchlässigkeit von mindestens 800 g/ m$^2$/ 24 Std. und vorzugsweise von mindestens 1000 g/ m$^2$/ 24 Std. (gemessen nach DIN EN 13726-2 (2002)) aufweisen.

Gemäß einer weiteren Ausführung ist es jedoch auch möglich, als Trägerschicht einer erfindungsgemäßen Wundauflage Faservliese einzusetzen. Diese Faservliese sind insbesondere aus hydrophoben Fasern gefertigt und mittels Wasserstrahlen verfestigt.

Es ist jedoch auch vorgesehen als Trägerschicht ein Meltblown- Faservlies, Spunlaced- Faservlies oder ein Airlaid- Faservlies zu verwenden, wobei diese Faservliese weiter bevorzugt aus Polyurethan, Polystyrol- Isopren- Blockcopolymeren, Polystyrol- Butadien- Blockcopolymeren, Polyester, Polyolefinen oder Polyesterphthalaten gefertigt werden.

Im Folgenden soll.die Erfindung an Hand einer Zeichnung erläutert werden, ohne dass diese Zeichnung eine bezüglich der Erfindung einschränkende Wirkung haben soll. Dabei zeigen:

Figur 1     eine Ausführung einer erfindungsgemäßen Wundauflage in Aufsicht.

Figur 2     Erfindungsgemäße Wundauflage im Querschnitt.

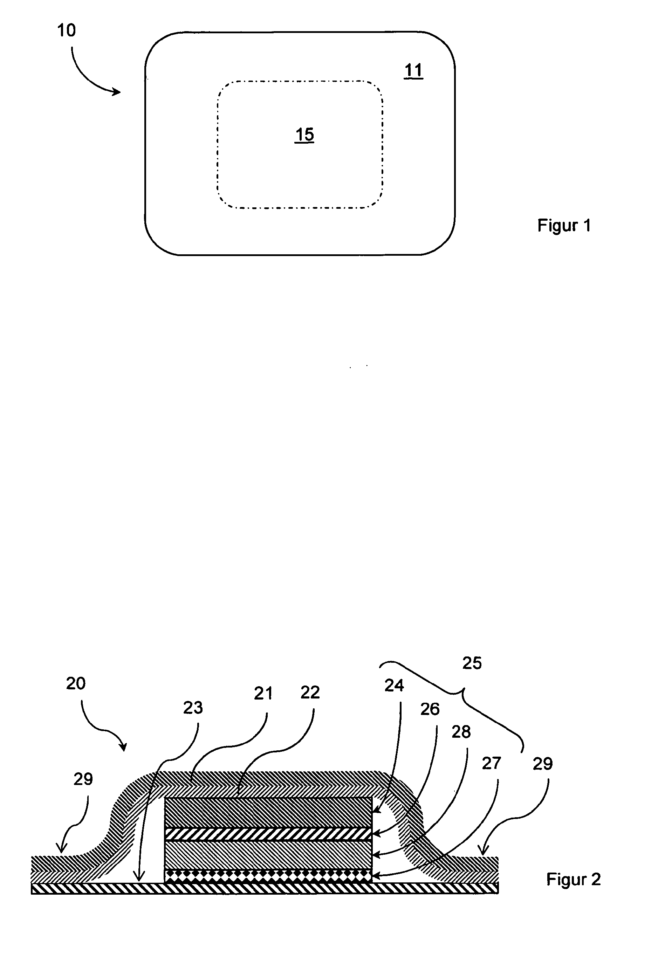

Mit Figur 1 ist eine erfindungsgemäße Wundauflage 10 in Aufsicht wiedergegeben. Die Wundauflage weist eine Trägerschicht 11 und ein absorbierendes Kissen 15 auf. Die Trägerschicht 11 ist einseitig auf der Rückseite vollflächig mit einem haufreundlichen Haftklebstoff beschichtet (nicht dargestellt). Das absorbierende Kissen 15 ist im Zentrum der Trägerschicht angeordnet, so dass ein klebender Rand zur Befestigung auf der Haut eines Patienten das absorbierende Kissen allseitig umgibt. Die Wundauflage 10 ist als so genanter Inselverband gefertigt.

Mit Figur 2 ist eine erfindungsgemäße Wundauflage 20 im Querschnitt gezeigt. Die Wundauflage besteht aus einer Trägerschicht 21 aus einem wasserabweisenden Faservlies, das mit einem Haftklebstoff 22 vollflächig beschichtet ist. Das Laminat bestehend aus dem Haftklebstoff 22 und der Trägerschicht 21 ist für Wasserdampf durchlässig, so dass eine atmungsaktive Wundauflage bereitgestellt wird. Zentrumssymmetrisch ist auf der Trägerschicht ein mehrschichtiges absorbierendes Kissen 25 festgelegt, wobei ein klebender Rand 29 zur Festlegung auf einem Körper verbleibt. Das absorbierende Kissen 25 besteht aus einer ersten absorbierenden Schicht 28 aus einem ersten absorbierenden Fasermaterial, einer zweiten absorbierenden Schicht 24 aus einem von dem ersten verschiedenen zweiten absorbierenden Fasermaterial, einer zwischen der ersten und der zweiten Schicht angeordneten dritten Schicht 26, die die erste und die zweite Schicht beabstandet Die dritte Schicht 26 besteht aus einer netzartigen Polyethylenfolie, die beidseitig mit elementarem Silber beschichtet ist. Die erste absorbierende Schicht 28 umfasst als absorbierendes Material ein vernadeltes Faservlies, das Viskosefasern umfasst. Die zweite absorbierende Schicht 24 umfasst als absorbierendes Fasermaterial ein Fasergemisch aus superabsorbierenden Polyacrylatfasern und Viskosefasern. Durch den Anteil an superabsorbierenden Polyacrylatfasern weist die zweite absorbierende Schicht 24 im Vergleich zu der ersten absorbierenden Schicht 28 eine wesentlich größere freie Absorption auf als die erste absorbierende Schicht. Des Weiteren weist das absorbierende Kissen eine für Wundexsudat durchlässige, nicht mit der Wunde verklebende Wundkontaktschicht 27 aus einer Polymerfolie aus Polyethylen auf. Durch den gewählten Schichtaufbau wird das Wundexsudat von der Wunde abgehalten und eine Mazeration der Wund und der umliegenden Wundränder verhindert. Durch die Anordnung der mit Silber beschichteten Polymerfolie zwischen den beiden absorbierenden Schichten kann die Wundauflage auch bei einer längeren Verweilzeit von drei bis fünf Tagen auf einer Wunde weitgehenst keimfrei gehalten werden, so dass eine Rekontamination der Wunde mit Keimen unterbunden wird. Die im anwendungsgerechten Zustand der Wundauflage dem Körper des Patienten zugewandte Seite ist zum Schutz vor der Verwendung mit einem ablösbaren Releasepapier 23 abgedeckt.

Ausführungsbeispiel

I) Testmethoden

1) Freie Absorption von Kochsalzlösung - Test 1

Die freie Absorption von physiologischer Natriumchlorid- Lösung (0, 9 Gew. %- ig, NaCl in Wasser) wird in Anlehnung an EN 13726- 1 (2002) bestimmt. Der einzige Unterschied zur DIN EN 13726- 1 (2002) ist die Verwendung von NaCl- Lösung anstelle von NaCl- und $CaCl_2$- Lösung, wobei folgendes gilt:

$$Flüssigkeitsaufnahme = \frac{M_2 - M_1}{M_1}[g/g]$$

(1)

M2 = Nassgewicht der Probe in g
M1 = Trockengewicht der Probe in g

2) Freisetzung Silber - Test 2

Zur Bestimmung der Freisetzung von Silber aus der Wundauflage wird eine Probe des absorbierenden Kissens (ohne die Trägerschicht) von 5 x 5 cm in einen mit 100 ml destilliertem Wasser gefüllten, verschließbaren Erlenmeierkolben gegeben, so dass das absorbierende Kissen vollständig von Wasser bedeckt ist. Die Probe wird in dem verschlossenen Kolben 24 Stunden bei 25 °C belassen. Nach 24 Stunden wird die Probe aus dem Kolben entfernt, die zurückbleibende Flüssigkeit filtriert und der Gehalt an Silber in der filtrierten Flüssigkeit mittels Atomabsorptionsspektroskopie bestimmt.

II) Aufbau der Wundauflage

Die Wundauflage weist im Wesentlichen den mit Figur 2 wiedergegebenen Aufbau auf, wobei im Folgenden zur Bezeichnung der einzelnen Bestandteile der Wundauflage dieselben Begriffe verwendet werden. Die Wundauflage besteht aus einem Trägermaterial aus einem wasserundurchlässigen, wasserstrahlvernadelten Faservlies aus Polyesterfasern mit einem Flächengewicht von 70 g/ m$^2$ (Sontara 8010, Du Pont) . Auf dem Trägermaterial ist ein auf Synthesekautschuk basierter Heißschmelzklebstoff in einer Menge von 35 g/ m$^2$ vollflächig aufgebracht. Die Wundauflage weist eine Größe von 10 x 8 cm auf, wobei das absorbierende Kissen (Typ P 2007/123/7/3; Fa. Kemex- AL Almelo, Niederlande) mit einer Größe 6 x 4 cm zentrumssymmetrisch auf den Haftklebstoff aufgebracht ist, so dass ein klebender Rand von 2 cm das absorbierende Kissen allseitig umgibt. Jede Schicht des absorbierenden Kissens ist bezüglich ihres äußeren Umfangs gleich groß wie eine zweite Schicht des absorbierenden Kissens. Das absorbierende Kissen weist eine nichtwundverklebende Wundkontaktschicht auf, auf die eine erste absorbierende Schicht aus einem vernadeltem Faservlies laminiert ist, das aus einem Fasergemisch aus Viskosefasern, Polyethylenfasern und Polypropylenfasern besteht. Die Wundkontaktschicht besteht aus einem microporösem Polyethylennetz mit einem Flächengewicht von 32 g/ m$^2$. Die Wundkontaktschicht weist eine Vielzahl an Öffnungen oder Durchbrechungen zum Durchtritt von Flüssigkeiten auf, wobei diese Öffnungen oder Durchbrechungen eine Fläche von ca. 20 % der Fläche der Wundkontaktschicht einnehmen. Das Faservlies der ersten absorbierenden Schicht weist ein Flächengewicht von 275 g/ m$^2$ und eine freie Absorption von 1965 g/ m$^2$ (gemessen nach DIN EN 13726- 1 (2002)- Test 1) auf. Damit beträgt die freie Absorption der ersten absorbierenden Schicht $A_1$ = 7, 1 g/ g. Des Weiteren weist das absorbierende Kissen ein Air- Laid- Faservlies als zweite absorbierende Schicht auf. Das Air- Laid- Faservlies besteht aus einem Fasergemisch aus 20 Gew.- % (bezogen auf das Air- Laid- Faservlies) superabsorbierenden Polyacrylatfasern aus einem Polyacrylat- Polymethacrylat- Copolymer, 75 Gew. % Viskosefasern und 5 Gew.- % thermoplastische Bikomponentenfasern aus Polyethylen und Polypropylen. Das Air- Laid- Faservlies weist ein Flächengewicht von 85 g/ m$^2$ und eine freie Absorption von 1800 g/ m$^2$ (gemessen nach DIN EN 13726- 1 (2002)- Test 1) auf. Damit beträgt die freie Absorption der zweiten absorbierenden Schicht $A_2$ = 21, 2 g/ g. Das Verhältnis $A_1$ zu $A_2$ beträgt damit 1 : 3. Zwischen der ersten und der zweiten absorbierenden Schicht ist ein einseitig mit elementarem Silber beschichtetes Polymernetz aus Polyethylen derart angeordnet, dass die Silberschicht zur ersten absorbierenden Schicht weist. Das beschichtete Polymernetz weist ein Flächengewicht von 20 g/ m$^2$ auf, wobei die Menge an elementarem Silber auf dem Polymernetz 150 bis 300 mg/ m$^2$ beträgt. Das Polymernetz weist eine Vielzahl an Öffnungen oder Durchbrechungen zum Durchtritt von Flüssigkeiten auf, wobei diese Öffnungen oder Durchbrechungen eine Fläche von ca. 50 % der Fläche des Polymernetzes einnehmen. Das Gesamtgewicht des absorbierenden Kissens beträgt 412 g/ m$^2$ wobei das absorbierende Kissen eine freie Absorption von 3765 g/ m$^2$ (gemessen nach DIN EN 13726- 1 (2002)- Test 1) aufweist. Damit weist das absorbierende Kissen eine freie Absorption $A_T$ von 9, 1 g/ g auf.

Die Wundauflage ist nicht als antimikrobielle Wundauflage zu bezeichnen, da die Freisetzungsrate an Silber aus der Wundauflage lediglich 11,3 µg Silber pro 1 g absorbierendes Kissen in 24 Std. bei 25 °C in 100 ml Wasser an Silber in elementarer Form oder in Form von Salzen oder Komplexen freisetzt, wobei zur Bestimmung der abgegebenen Menge Silber die Atomabsorptionsspektroskopie herangezogen wurde. Somit wirkt das antimikrobielle Mittel fast ausschließlich innerhalb der Wundauflage. Bei der bestimmungsgemäßen Verwendung dieser Wundauflage unterbleibt somit eine Belastung an zusätzlichen Wirkstoffen auf und in der Wunde.

**Patentansprüche**

1. Wundauflage (20) zur Behandlung von mäßig bis stark exsudierenden Wunden umfassend eine Trägerschicht (21) und ein absorbierendes Kissen (25), wobei das absorbierende Kissen (25) eine erste absorbierende Schicht (28) umfassend ein erstes absorbierendes Material und eine von der ersten Schicht verschiedene zweite absorbierende Schicht (24) umfassend ein von dem ersten Material verschiedenes zweites absorbierendes Material umfasst, und wobei die erste Schicht (28) von der zweiten Schicht (24) durch mindestens eine dritte Schicht (26) beabstandet ist, wobei diese dritte Schicht (26) mindestens ein antimikrobielles Mittel umfasst, **dadurch gekennzeichnet, dass** das zweite absorbierende Material superabsorbierende Fasern umfasst und dass das absorbierende Kissen (25) weiterhin eine Wundkontaktschicht (27) umfasst, die mit der ersten Schicht (28) in unmittelbaren Kontakt steht, wobei die Wundkontaktschicht (27) ein Polymernetz oder eine perforierte Polymerfolie ist.

2. Wundauflage (20) nach Anspruch 1 **dadurch gekennzeichnet, dass** das erste absorbierende Material ein Fasermaterial ist.

3. Wundauflage (20) nach mindestens einem der vorgenannten Ansprüche **dadurch gekennzeichnet, dass** die zweite absorbierende Schicht (24) eine größere freie Absorption aufweist als die erste absorbierende Schicht (28), *gemessen nach DIN EN 13726-1 (2002).*

4. Wundauflage (20) nach mindestens einem der vorgenannten Ansprüche **dadurch gekennzeichnet, dass** das Verhältnis der freien Absorption $A_1$ der ersten absorbierenden Schicht zu der freien Absorption $A_2$ der zweiten absorbierenden Schicht derart eingestellt ist, dass das Verhältnis $A_1 : A_2$ dem Verhältnis von 1 : 2 bis 1 : 10 entspricht, *gemessen nach DIN EN* 13726-1 *(2002).*

5.  Wundauflage (20) nach mindestens einem der vorgenannten Ansprüche **dadurch gekennzeichnet, dass** die dritte Schicht (26) ein Polymernetz oder perforierte Polymerfolie ist, die mit einem antimikrobiellen Mittel beschichtet ist.

6.  Wundauflage (20) nach mindestens einem der vorgenannten Ansprüche **dadurch gekennzeichnet, dass** das antimikrobielle Mittel elementares Silber, ein Silberoxid, ein Silberkomplex oder ein Silbersalz oder Mischungen hiervon umfasst.

7.  Wundauflage (20) nach mindestens einem der vorgenannten Ansprüche **dadurch gekennzeichnet, dass** die Wundkontaktschicht (27) und/ oder die erste absorbierende Schicht (28) keine antimikrobiellen Mittel umfasst.

8.  Wundauflage (20) nach mindestens einem der vorgenannten Ansprüche **dadurch gekennzeichnet, dass** die Trägerschicht (21) aus einem Faservlies oder einem Polymerfilm besteht.

## Claims

1.  Wound dressing (20) for treating strongly or moderately exudating wounds comprising a backing layer (21) and an absorbent pad (25), wherein the absorbent pad (25) comprises a first absorbent layer (28) comprising a first absorbent material and a second absorbent layer (24) which is other than the first layer and comprises a second absorbent material other than the first material, and wherein the first layer (28) is distanced from the second layer (24) by at least one third layer (26), wherein this third layer (26) comprises at least one antimicrobial agent, **characterized in that** the second absorbent material comprises superabsorbent fibers and **in that** the absorbent pad (25) further comprises a wound contact layer (27) which is in immediate contact with the first layer (28), wherein the wound contact layer (27) is a polymer net or perforated polymer foil.

2.  Wound dressing (20) according to Claim 1 **characterized in that** the first absorbent material is a fiber material.

3.  Wound dressing (20) according to at least one of the aforementioned claims **characterized in that** the second absorbent layer (24) has a greater free absorbency than the first absorbent layer (28), measured to DIN EN 13726-1 (2002).

4.  Wound dressing (20) according to at least one of the aforementioned claims **characterized in that** the ratio of the free absorbency $A_1$ of the first absorbent layer to the free absorbency $A_2$ of the second absorbent layer is such that the $A_1:A_2$ ratio corresponds to the ratio in the range from 1:2 to 1:10, measured to DIN EN 13726-1 (2002).

5.  Wound dressing (20) according to at least one of the aforementioned claims **characterized in that** the third layer (26) is a polymer net or perforated polymer foil coated with an antimicrobial agent.

6.  Wound dressing (20) according to at least one of the aforementioned claims **characterized in that** the antimicrobial agent comprises elemental silver, a silver oxide, a silver complex or a silver salt or mixtures thereof.

7.  Wound dressing (20) according to at least one of the aforementioned claims **characterized in that** the wound contact layer (27) and/or the first absorbent layer (28) comprises no antimicrobial agents.

8.  Wound dressing (20) according to at least one of the aforementioned claims **characterized in that** the backing layer (21) consists of a fibrous nonwoven web or a polymer film.

## Revendications

1.  Pansement (20) pour le traitement de plaies exsudant moyennement à fortement, comprenant une couche support (21) et un coussin absorbant (25), le coussin absorbant (25) comprenant une première couche absorbante (28) comprenant un premier matériau absorbant et une deuxième couche absorbante (24) différente de la première couche absorbante, comprenant un deuxième matériau absorbant différent du premier matériau et la première couche (28) étant écartée de la deuxième couche (24) par au moins une troisième couche (26), cette troisième couche (26) comprenant au moins un agent antimicrobien, **caractérisé en ce que** le deuxième matériau absorbant comprend des fibres superabsorbantes et **en ce que** le coussin absorbant (25) comprend en outre une couche de contact (27) avec la plaie, qui est en contact direct avec la première couche (28), la couche de contact (27) avec la plaie étant une toile polymère ou une feuille polymère perforée.

2. Pansement (20) selon la revendication 1, **caractérisé en ce que** le premier matériau absorbant est un matériau fibreux.

3. Pansement (20) selon au moins l'une quelconque des revendications susmentionnées, **caractérisé en ce que** la deuxième couche absorbante (24) présente une plus grande absorption libre que la première couche absorbante (28), mesurée selon la norme DIN EN 13726-1 (2002).

4. Pansement (20) selon au moins l'une quelconque des revendications susmentionnées, **caractérisé en ce que** le rapport de l'absorption libre $A_1$ de la première couche absorbante à l'absorption libre $A_2$ de la deuxième couche absorbante est réglé de manière telle que le rapport $A_1:A_2$ correspond au rapport 1:2 à 1:10, mesuré selon la norme DIN EN 13726-1 (2002).

5. Pansement (20) selon au moins l'une quelconque des revendications susmentionnées, **caractérisé en ce que** la troisième couche (26) est une toile polymère ou une feuille polymère perforée, qui est revêtue par un agent antimicrobien.

6. Pansement (20) selon au moins l'une quelconque des revendications susmentionnées, **caractérisé en ce que** l'agent antimicrobien comprend de l'argent élémentaire, un oxyde d'argent, un complexe d'argent ou un sel d'argent ou des mélanges de ceux-ci.

7. Pansement (20) selon au moins l'une quelconque des revendications susmentionnées, **caractérisé en ce que** la couche de contact (27) avec la plaie et/ou la première couche absorbante (28) ne comprend pas d'agent antimicrobien.

8. Pansement (20) selon au moins l'une quelconque des revendications susmentionnées, **caractérisé en ce que** la couche support (21) est constituée par un non-tissé en fibres ou un film polymère.

Figur 1

Figur 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19727032 A1
- US 5465735 A
- US 5632731 A
- WO 9841095 A1
- WO 0160599 A1
- WO 2004112852 A1
- DE 10108083 A1